# EUROPEAN PATENT APPLICATION

(11) **EP 4 821 542 A2**
(43) Date of publication of application: **14.10.2026**
(21) Application number: 26202452.4
(22) Date of filing: 15.02.2022
(51) Int. Cl.: A61N 1/378

(54) **ORIENTATION DEPENDENT REFORMING**

(30) Priority: 01.03.2021 EP 21159940
(62) Document number(s) of the earlier application(s) in accordance with Art. 76 EPC: 22705781.7 / 4 301 458
(71) Applicant: BIOTRONIK SE & Co. KG, 12359 Berlin (DE)
(72) Inventor: Dorr, Thomas, 12437 Berlin (DE)
(74) Representative: Biotronik Corporate Services SE

(57) **Abstract**

The present disclosure relates to an implantable defibrillator which comprises at least one energy source and at least one electrical component. The implantable defibrillator further comprises means for activating a coupling between the energy source and the electrical component, based at least in part on an orientation and/or a motional state of the energy source, wherein the electrical component is a capacitor.

## Description

The present invention generally relates to an implantable defibrillator and methods and computer programs for operating such defibrillator, particularly to an activation of a coupling based on an orientation and/or a motional state of an energy source of the defibrillator.

An implantable cardioverter-defibrillator (ICD) is a device, which is or can be implanted into a patient for detecting and treating abnormal heartbeats. Its main applications are cardioversion, the treatment of abnormal cardiac arrhythmias, as well as defibrillation, the treatment of life-threatening cardiac arrhythmias.

Common ICDs may comprise a casing implanted in the upper body of the patient comprising a computing unit, a battery and a high-voltage capacitor. A transvenous electrode-system may be connected to the heart and may include sensors for heartbeat monitoring.

If a critical heartbeat is detected by the ICD, it delivers controlled electric pulses over the electrode-system to the heart to normalize the cardiac arrythmia. For a successful treatment, a defined electrical energy needs to be delivered to the heart. This may be realized with the capacitor of the ICD, which temporarily stores electrical energy from the battery and releases it in a controlled way. During application the capacitor is charged, e.g. over charging circuitry, with a subsequent release of the electrical energy with an electric pulse typically in the millisecond range. The electric pulses may be set up to have a certain shape and may be timed to the cardiac cycle.

During application, battery power is consumed by the electrical components of the ICD in various ways. Besides, stress resulting from operation of the battery can lead to degradation effects reducing battery lifetime or even a sudden destruction of the battery. A replacement of the battery can typically only be done by surgery causing medical stress on the patient such that frequency of the need for battery replacement should be minimized.

The currently known techniques for operating implantable defibrillators do not always lead to an optimum battery lifetime. Therefore, there is a need to find ways to improve battery lifetime.

The aspects described herein address the above need.

A first aspect relates to an implantable defibrillator which comprises at least one energy source and at least one electrical component. The implantable defibrillator further comprises means for activating a coupling between the energy source and the electrical component, based at least in part on an orientation and/or a motional state of the energy source, wherein the electrical component is a capacitor.

An underlying idea of the present invention is that a major limitation of prior art approaches is caused by the fact that these do not take into account the state of the energy source (e.g. battery, e.g. a lithium-ion battery) of an implantable defibrillator during the coupling to an electrical component. Once implanted, the orientation and/or motional state of the energy source corresponds at least in part to the orientation and/or motional state of the patient which has the defibrillator implanted into his/her body. The patient as a human being can put himself in many degrees of physical states that result in certain forces or conditions taking effect on the body and the implanted defibrillator comprising the energy source. The patient could be in various positions or orientations, for example standing up, sitting upright, laying down, doing a handstand or an arbitrary yoga position. Further the patient could be in different motional states, for example walking, taking part in athletic activities, e.g. running, jumping, diving, figure skating. A motional state could also be inflicted on the patient by being in a moving vehicle, for example a car, a plane or a rollercoaster. Depending on the condition, the exerted forces are not negligible on the energy source.

For example, the risk of leakage from the energy source when coupling it to the electric component such that current flows may be dramatically increased when the energy source is strongly tilted (e.g. when the patient lays down or even stands upside down), and such leakage can be detrimental for the lifetime of the energy source. If coupling in such an orientation of the energy source is avoided, its lifetime can correspondingly be dramatically increased. This particularly applies, if large amounts of current are expected to flow and/or if currents are expected to flow over a prolonged period of time from the energy source via the coupling. Similar considerations may apply if the energy source is in a state of strong motion (e.g. when the patient is running), and hence avoiding coupling in such state may also be beneficial to maximize lifetime. The present invention ensures that the orientation and/or motional state of the energy source are taken into account, when coupling it to the electrical component, such that it may be ensured that coupling only occurs when the orientation and/or motional state are not adversely affecting the lifetime of the energy source, when coupling.

Since the battery is typically fixedly connected to the remaining parts of the defibrillator (which in turn is typically fixedly connected to a patient), the orientation and motional states of the battery may be understood to be associated with those of the defibrillator and the patient. Hence, instead of using the orientation and/or motional state of the energy source, as mostly described herein, the aspects as described herein are also applicable to the orientation and/or motional state of the defibrillator itself as well as of the (upper body of the) patient.

It is noted that an implantable defibrillator is understood herein as any implantable device that may provide defibrillation and/or cardioversion, for example an implantable cardioverter-defibrillator (ICD), a subcutaneous implantable cardioverter-defibrillator (S-ICD), an extravascular implantable cardioverter-defibrillator (EV-ICD), or a cardiac resynchronization therapy (CRT) device, which may comprise an ICD, e.g. a CRT-D.

For example, the means for activating may include a switch and/or a (charging) circuitry and/or a control unit (possibly adapted to operate a switch or other (charging) circuitry between energy source and electrical component). The electrical component may be a capacitor or any other electrical component of the defibrillator.

If the electrical component is a capacitor of the defibrillator, the coupling may for example be arranged such as to reform the capacitor, charge the capacitor, and/or a calibration or testing procedure. Particularly, coupling the energy source to a capacitor typically leads to a relatively high current flow over prolonged periods of time, such that ensuring a proper state of the energy source before such coupling allows avoiding particularly detrimental effects on the lifetime of the energy source.

According to an example, the defibrillator may further comprise means for determining the orientation and/or motional state of the battery. Hence, the defibrillator itself may verify that the battery has a beneficial orientation and/or motional state, based on which the electrical coupling may be activated. The defibrillator may thus, in some examples, operate in an autarkic manner. The means for determining the orientation and/or motional state of the battery may comprise one or more sensors. For example, the means for activating (e.g. control unit) may be coupled to receive data from the one or more sensors to determine the orientation and/or motional state.

For example, the means for determining may comprise or be implemented by at least one of the following: an orientation sensor, a position sensor, an activity sensor, a tilt sensor, an inertial sensor, an acceleration sensor, a rotational sensor, an inertial measurement unit (IMU), an inertial navigation system (INS), or a 3D-sensor. An activity sensor could be implemented by or include an accelerometer and/or a piezo sensor detecting body vibrations. An inertial sensor may include or be implemented by an accelerometer and/or a sensor detecting a rate of rotation, for example.

The means for activating may comprise a control unit wherein a sensor may be coupled to the control unit for acquiring sensor data. The control unit may be configured to handle sensor data processing, e.g. filtering of sensor data, feature extraction, further calculations, etc., to determine the orientation and/or motional state.

In a further example the defibrillator may be configured to activate the electrical coupling, based at least in part on the determined orientation deviating from a set orientation value by less than a first predetermined threshold and/or the determined motional state deviating from a set motional state value by less than a second predetermined threshold. The set orientation value, the set motional state value and/or the predetermined thresholds could be based on battery type, defibrillator type, patient data, such as patient age. In some examples, the defibrillator (e.g. the means for activating) is programmable with one or more set values and/or threshold values.

For example, an orientation could be a tilt of the energy source. In an example, the activation of the electrical coupling could be allowed when a tilt of the energy source from a "normal" orientation (e.g. battery source and/or patient stand fully upright; along z-axis as understood herein) is below a certain threshold angle (or it could be inhibited if the tilt is above the threshold). It should be mentioned that the tilt may not merely be determined by the absolute value of the tilt angle. The tilt direction, e.g. the rotational direction, could also be considered. This could be beneficial to alleviate battery degradation effects which may depend on tilt direction. Threshold values for a tilt angle (from "normal" orientation) could for example be 135°, below 90°, below 45°, below 40°, below 30°, below 20°, or below 10°, with or without consideration of the tilt direction.

It may also be possible to use anisotropic thresholds depending on the axis of rotation of the energy source. In an example, the activation of the electrical coupling could be allowed when a first tilt of the energy source along a first horizontal axis (e.g. perpendicular to a "normal" orientation; e.g. an x-axis) is below a first threshold angle and/or a second tilt along a second horizontal axis (e.g. perpendicular to the first horizontal axis; e.g. a y-axis) is below a second threshold angle. The same exemplary thresholds as outlined above may apply. In another example, an activation could be inhibited if at least one of the first and second tilts is above its respective threshold. Further it is possible that a tilt with respect to another axis, e.g. a vertical axis (parallel to the "normal" orientation), would not be relevant to the activation or would have a different threshold than the threshold angle(s) of the tilt with respect to horizontal axis (or axes). The "normal" orientation may be energy source specific. For example, it may be adapted to correspond to the patient standing upright. However, also different implementations are possible.

Additionally or alternatively, in a further example, a motional state may be determined by a sensor signal which may comprise one or more motional state signals along one, two, three or more axes and/or rotation directions. For example, the sensor signal may comprise acceleration, rotational, or activity values along one or more axes and/or rotational directions.

The overall motional state may be considered, by determining a mean value of the motional state values along all respective axes (or rotational directions) and comparing the mean value to a predetermined threshold value (or only a single axis or rotational direction is considered in the first place). For example, the coupling could be activated based on the (mean) value of the acceleration and/or rotation being below a predetermined threshold.

The coupling could also be based on conditions of the motional state specific to certain axes or directions. With this concept a wide degree of various motional states can be covered for activation or inhibition of the coupling. The conditions may depend on the battery type, implantable defibrillator type and patient data. In an example, the activation of the coupling may be allowed if the value (e.g. rotation or acceleration) along each axis and/or each rotation direction is below a predetermined threshold (that may be specific to that axis or rotation direction). The coupling may also be inhibited as long as a value along at least one axis/direction is above a predetermined threshold. Also here, different thresholds may be used for different axes/directions. It may also be, that at least one axis or rotational direction is not relevant for determining the motional state, and thus may be omitted from determining the motional state.

In another example the motional state may be determined based on an activity signal, e.g. from an activity sensor, such as a piezo sensor. For example, the activity signal may correlate to body vibrations. The coupling may be allowed or inhibited if the activity signal indicates an activity below or above a predetermined threshold, which may correspond to a critical intensity of activity.

In a further example, the type of athletic activity of the patient or if the patient is in a moving vehicle etc. could be determined as a motional state. The activation of the electrical coupling may be allowed or inhibited regarding a predetermined critical athletic activity or vehicle movement. For example, coupling could be inhibited if the motional state indicates movement in a car or heavy athletic activity, since then uncontrolled events may be expected (due to sudden braking or acceleration, for example).

In some examples, the means for coupling may be adapted to activate (or inhibit) the coupling based at least in part on the determined orientation and/or motional state being below (or above) threshold (cf. above) for a predetermined duration. For example, a condition for activating may be that the determined values are below threshold (not above threshold) for, e.g., at least 10 seconds, at least one minute, at least five minutes, at least ten minutes, at least 30 minutes, etc., which may indicate that the patient (and correspondingly the energy source) indeed rests in a beneficial orientation and/or motional state for a prolonged period of time.

In another example, the means for activating the electrical coupling may be further configured to estimate the orientation and/or motional state of the electrical energy source based on a time, e.g. a time of the day. For example, the coupling may be activated if the time of the day indicates a beneficial orientation and/or motional state. This may, for example be in a specific night time (e.g. between 1 and 4 am), wherein the patient may be estimated to be sleeping in a laying position. Alternatively, this may for example be during a specific morning time (e.g. between 9 and 11 am), wherein the patient may be estimated to be in a sitting position such that the upper body is upright. For example, this may be beneficial if the defibrillator is implanted such that an upright body corresponds to a favorable battery position. The time could be provided by a time unit of or coupled to the means for activating. Additionally or alternatively, the time may be provided by a signal received from an external source (e.g. a time signal provided by a mobile device, smartphone, smartwatch, or a medical device etc.) by corresponding means for receiving of the implantable defibrillator (e.g. based on Bluetooth, NFC, WiFi, 4G, 5G, or any other suitable means). The estimation may be patient-specific, e.g., for patients working night shifts, it may not be suitable to estimate a laying position based on a night time. It is also possible that the estimation is based on a calendar of the patient (e.g. as stored on a smartphone), such that the defibrillator may estimate the orientation and/or motional state based on the time and the calendar (the latter e.g. provided by the smartphone). For example, times during which sporting activities are scheduled may be avoided.

The means for activating may be configured to activate the coupling, at least in part, based on the estimated orientation deviating from a set orientation value by less than a first predetermined threshold; and/or the estimated motional state deviating from a set motional state value by less than a second predetermined threshold. Here, the same principles and algorithms may be implemented as outlined herein with respect to the determined orientation and/or the determined motional state (e.g. as determined based on sensor data).

In another example, the defibrillator may comprise means for receiving information about the orientation and/or motional state from an external source. The defibrillator further may optionally comprise means to transmit information to the external source. The means for receiving and/or transmitting information could be one or more receiver, transmitter or transceiver units arranged in the defibrillator (e.g. based on NFC, Bluetooth, WiFi, 4G, 5G etc. as outlined above). As an external source many options are possible such as a mobile device, smartphone, smartwatch, fitness tracker, a security camera, a smart home device, or a medical device, etc. The external source may comprise means to determine the orientation and/or motional state of the energy source and/or patient. It may further be configured to periodically transmit the determined orientation and/or motional state to the defibrillator. Additionally or alternatively, it may send these to the defibrillator upon request by the defibrillator. It is also possible that the external source estimates a motional state and/or orientation (e.g. based on a calendar) and corresponding information may then be transmitted to the defibrillator.

The means for activating may be configured to determine and/or estimate the orientation and/or motional state, at least in part, based on the information received from the external source (in addition to or as an alternative to a determination based on data received from one or more sensor(s) of the defibrillator; for example the orientation and/or motional state may be determined based on both internal and external data sources). The means for activating may further be configured to activate the coupling, at least in part, based on the determined orientation deviating from a set orientation value by less than a first predetermined threshold; and/or the estimated motional state deviating from a set motional state value by less than a second predetermined threshold. Here, the same principles and algorithms may be implemented as outlined herein with respect to the orientation and/or the motional state determined e.g. based on sensor data and/or estimated based on a time.

In an example, the energy source of the defibrillator may comprise a first portion which is at least partially filled with liquid (e.g. liquid electrolyte), and a second portion which comprises at least one connection for the coupling. The means for activating may be configured to activate the coupling, based on the orientation and/or motional state of the energy source being such, that a flow of liquid from the first portion to the second portion is suppressed (and/or that a pressure of the liquid onto the second portion is reduced). Hence, it may be avoided that liquid flows into the second portion (which may not be provided to comprise or contain liquid) which could harm the at least one connection. The second portion may in particular be adjacent to the first portion.

The first portion may comprise an anode-cathode packet whereas the liquid of the first portion may comprise an electrolyte. The second portion may be hollow and/or partly filled with a solid filler material. If the energy source has an unfavorable orientation (e.g. such that the first portion is above the second portion such that gravity drives the liquid onto/into the second portion) or an unfavorable motional state (e.g. such that the liquid is shaken within the first portion such that waves of the liquid may impinge onto the second portion), liquid flow from the first portion to the second portion may be favored, possibly resulting in the formation of a liquid drop in/at the second portion. Under coupling conditions, particularly high power coupling, material residues of the liquid drop may remain on the edges of the first portion, inside the second portion and/or on the connection for the coupling located in the second portion. For example, when applying a current, in particular a high current, a relatively high ion concentration may be induced in the electrolyte drop in the second portion causing (permanent) ion material deposition after the coupling has stopped. The material residues may form unwanted electrical connections leading to electrical shorts causing internal leakage currents resulting in premature battery degradation. By suppressing liquid flow from the first portion to the second portion, this life shortening mechanism is actively eliminated. The energy source orientation and/or motional state in which said liquid flow is suppressed may be energy-source-specific and/or patient-specific. For example, the implantable defibrillator may be programmed with a desired patient- and/or energy-source-specific set orientation.

In another example, the means for activating may further be configured to activate the coupling based on the second portion being in an upper part of the energy source. Due to the principle of gravitational forces, this inhibits liquid to flow from the first portion to the second portion, thus alleviating the outlined life shortening mechanism. For example, the second portion may form or at least in part be level with a topmost part of the energy source (along the direction of gravity; z-axis as understood herein). For example, the center of gravity of the second portion may be above the center of gravity of the first portion and/or of the energy source.

In a further example, the means for activating may further be configured to activate the coupling, based at least in part on a predetermined timing (that may be independent from a time of the day). Certain electrical components may, e.g., require a reoccurring, e.g. regular or periodic coupling to the energy source. It is therefore beneficial to trigger the coupling after a certain time has passed after the last coupling. This process may be automated to ensure autarkic application of the defibrillator without specific technical supervision. The coupling to the energy source may be triggered after a certain timespan has passed (but additionally only activated based on the orientation and/or motional state of the energy source as outlined above). The (regular) activation may thus additionally be based on a beneficial orientation and/or motional state of the energy source in which battery degradation is inhibited. This combines the benefits of an automated coupling process with an increased battery life. The timing may be provided by an internal timer of the defibrillator, e.g. a time unit. Additionally or alternatively, it may be provided by a timing signal supplied by an external source, e.g. a smartphone, etc. as described herein.

In another example, the electric component of the defibrillator comprises or is at least one (high-voltage) capacitor configured to deliver defibrillator pulses and/or cardioverter pulses. The implantable defibrillator may further be configured such that the activating of the coupling reforms the capacitor. Reforming may be understood as charging the capacitor to its maximum voltage. The capacitor may be a high-voltage capacitor delivering electrical pulses for heart treatment. To ensure a repeatable generation of the same predetermined electrical pulses over time, the electrical properties of the high-voltage capacitor may generally need to be kept in a defined specification range. Since a capacitor may be prone to degradation effects during application, counteracting means may be necessary to ensure stable electrical characteristics. The high-voltage capacitor of a defibrillator may comprise an electrolytic capacitor, where a degraded oxide layer serving as the dielectric medium may be regenerated by a reforming process without exchanging the capacitor. During the reforming process, specific electrical conditions are applied onto the capacitor which trigger (at least partial) anodic oxidation at the dielectric interface causing (at least partially) a reformation of the oxide layer. The reforming process puts high electrical stress on the battery which deviates significantly from normal power consumption. Particularly in this period, a disadvantageous battery orientation and/or motional state can lead to irreversible damage of the battery and/or battery interconnects and/or the defibrillator. The aspects described herein may minimize such damage.

A second aspect relates to a method for operating an implantable defibrillator. The method may comprise activating a coupling between an energy source and an electrical component of the implantable defibrillator, based at least in part on an orientation and/or a motional state of the energy source, wherein the electrical component is a capacitor. The coupling may be implemented by means for activating, such as a control unit comprising a switch and/or a charging circuitry for coupling.

In another example, the method furthermore comprises determining the orientation and/or the motional state of the energy source (and/or the defibrillator and/or the patient). Further a deviation of the determined orientation and/or motional state from a set value may be evaluated and compared to a predetermined threshold as outlined herein.

A further example of the method comprises estimating the orientation and/or the motional state of the energy source based on a time.

In another example, the method comprises receiving information about the orientation and/or the motional state of the energy source from an external source.

According to a third aspect, a computer program is provided comprising instructions to perform one of the methods as described herein. In an example the computer program instructions are stored on a non-transitory medium. For example, the computer program may be stored on an implantable defibrillator as described herein, and the latter may comprise means to execute the computer program instructions. The computer program may allow an autarkic, automated implementation of the aspects described herein. Consequently, technical intervention from medical staff and the patient may be minimized.

Whether described as method steps, computer program and/or means, the functions described herein may be implemented in hardware, software, firmware, and/or combinations thereof. If implemented in software/firmware, the functions may be stored on or transmitted as one or more instructions or code on a computer-readable medium. Computer-readable media include both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor. The control unit as described herein may also be implemented in hardware, software, firmware, and/or combinations thereof, for example, by means of one or more general-purpose or special-purpose computers, and/or a general-purpose or special-purpose processors.
- Fig. 1: Schematic two-dimensional cross-section of an exemplary energy source in various exemplary orientations and conditions.
- Fig. 2: Schematic representation of an exemplary embodiment of an implantable defibrillator according to the present invention.

Possible embodiments of the present invention will be described in the following. For brevity, only a few embodiments can be described. The skilled person will recognize that the specific features described with reference to these embodiments may be modified and combined differently and that individual features may also be omitted if they are not essential. The general explanations in the sections above will also be valid for the following more detailed explanations.

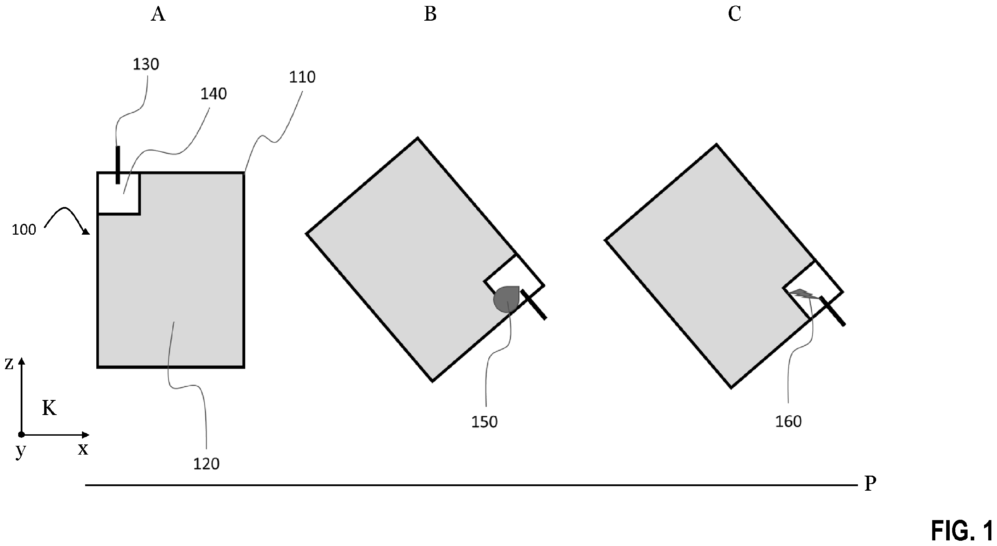

Fig. 1 shows, in various orientations and conditions, a two-dimensional schematic cross-section of an exemplary energy source 100. The exemplary energy source 100 may be built into an implantable defibrillator.

In this example, the energy source 100 is a battery. The battery may comprise a casing 110 that may be metallic and that may hermetically seal the battery. The battery may further comprise a first portion 120 that may be arranged within the casing 110 and that may comprise one or more or a multitude of anode-cathode-pairs. The anode-cathode-pairs may be soaked with and/or immersed in a liquid electrolyte. The battery may be a lithium-ion battery and the liquid electrolyte may, *inter alia*, comprise lithium ions. Each of the anode-cathode-pairs may be enclosed in separator foil. First portion 120 may arranged in the hermetically sealed metallic casing 110.

The battery may comprise a second portion 140 (e.g. a free volume) that may not be filled with liquid, in particular not with electrolyte or liquid electrolyte. The second portion may at least partly be enclosed by casing 110 or it may be provided separately and/or adjacent to the casing 110. At the second portion 140, one or more connections 130 (e.g. anode connector(s) and/or cathode connector(s)) may be provided. The connections 130 may lead from the inside of the second portion 140 to the outside of the metallic casing 110 of the battery to enable an external connection that allows current to flow from the battery to an electrical component. The second portion 140 may at least partially be surrounded (e.g. directly adjacent) by the first portion 120. For example, an essentially cuboid casing 110 may be provided, wherein the majority of its volume is comprised by the (liquid filled) first portion 120. A minority of its volume, preferably at a corner of the cuboid may not be comprised by the first portion 120, but by the second portion 140, instead. The casing 110 may comprise one or more openings around the second portion 140, such that the one or more connections 130 can be provided as external connection. The casing 110 may comprise a portion that hermetically seals the interface between first portion 120 and second portion 140.

Fig. 1 shows the energy source 100 in different orientations A, B, C. The orientations are relative to a plane P spanning along the x and y axes of the coordinate system K, wherein z may be a surface normal, e.g. represent the direction of the gravitational force of the earth. The terms such as up, down, upper, lower, etc. may thus be understood as relative to the z-axis.

The orientation A corresponds to an upright battery orientation, e.g. perpendicular to the plane P or parallel to the z-axis. The orientation may be understood as static, e.g., with no significant battery movement or motion. In battery orientation A the second portion 140 is in the upper part of the battery. The center of gravity of the second portion 140 is situated above the center of gravity of the first portion 120 and the battery itself (when referring to the center of gravity, a mere geometric understanding is assumed herein). The second portion 140 even can be considered as a top-most portion of the battery, e.g., not other portion of the battery is arranged at a higher position than second portion 140 (along the z-axis).

In orientation A, no significant amount of liquid electrolyte is contained in the second portion 140, e.g. due to the sealing provided between first portion 120 and second portion 140. The battery may safely supply high power, e.g. large amounts of currents or currents over a prolonged time, to an electrical component. Particularly, a capacitor may be safely reformed in orientation A of the battery.

In battery orientation B of Fig. 1, the battery is tilted with respect to the z-axis. Specifically, the battery is exemplarily rotated around the y-axis by about 135° from the upright orientation of orientation A. The second portion 140 is thereby in a lower part of the battery.

The center of gravity of the second portion 140 is situated below the center of gravity of the first portion 120 and the battery itself. The second portion 140 is no longer a top-most portion of the battery. At least a part of the first portion 120 containing the liquid electrolyte may be above at least a part of the second portion 140.

The inventors have found out that in such (or similar) orientations, due to gravitational forces, the liquid electrolyte may exert a higher pressure on the second portion. Despite a hermetically sealing of the interface between first portion 120 and second portion 140 (e.g. sidewalls (e.g. of the casing 110) between second portion 140 and first portion 120), this may then lead to an increased risk that a small quantity of liquid electrolyte enters the second portion 140, e.g. forming an electrolyte drop 150, therein.

If, under these conditions, the battery supplies electrical power to an electrical component, for example a capacitor, over the connector 130, the connector 130 and/or other components of the battery may be damaged.

This is shown in orientation C, which shows the same orientation as orientation B. Here, the result of a reforming process of a capacitor of an implantable defibrillator is shown, that was performed with the battery being in the battery orientation B with an electrolyte drop 150 in the second portion 140. It has been found that a current flow, particularly a high current flow such as during reforming, leads to a high ion-concentration in the electrolyte drop 150. Depending on battery type, the ions for example could be lithium ions from the lithium anode. Since the high ion-concentration may not vanish after the reforming process, this may result in a concentration gradient leading to unwanted material residues based on the ion type, e.g. lithium residues, which can accumulate at the edges of the anodes, e.g. lithium anodes. These may accumulate over time. Consequently, this may lead to parasitic electrical connections 160 between the anodes and metal components that are on the cathode potential. The electrical connections 160 may then cause internal leakage currents, leading to premature battery degradation.

A similar effect may be expected if the battery undergoes heavy motion. Also this may increase the risk of an electrolyte droplet 150 being formed in the second portion 140.

The present disclosure allows avoiding the above degradation mechanism identified by the inventor, namely by activating a coupling of the battery to an external electrical component based on an orientation and/or a motional state of the battery. For example, a reforming process may be performed when the battery is in an orientation and/or motional state such that the chances of electrolyte drop 150 formation are minimal.

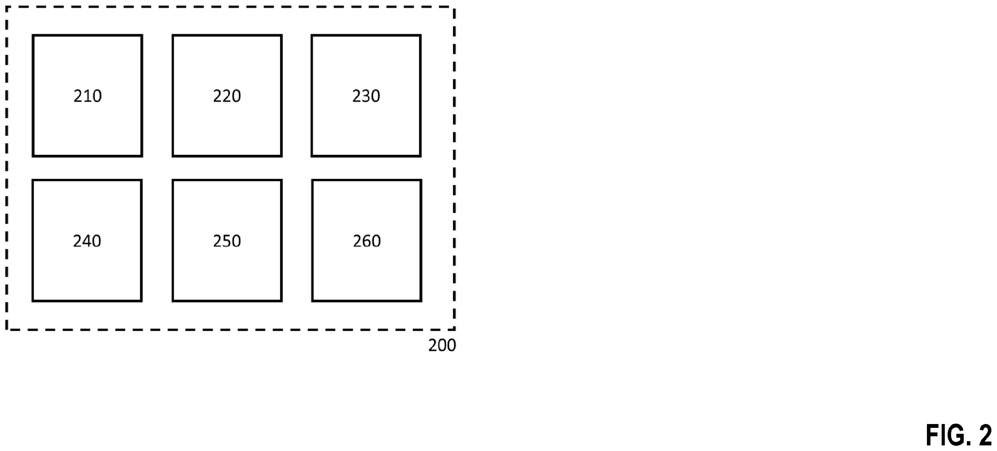

Fig. 2 shows a schematic representation of an example of an implantable defibrillator 200 according to the present invention. The implantable defibrillator 200 may be an implantable cardioverter-defibrillator (ICD). It may comprise a battery 210 and an electrical component, e.g. in the form of a high-voltage capacitor 220. It may further comprise a coupling between the battery 210 and the electrical component, e.g. in the form of a charging circuitry 230 for the high-voltage capacitor 220. It may further comprise a control unit 240, a time unit 250 and one or more sensors 260. The sensors 260 may comprise one or more orientation sensors 260 (e.g. to determine a tilt of the battery 210 with respect to the z-axis; and/or to determine relative rotations of the battery 210 around x- and y-axes, as outlined with respect to Fig. 1). Additionally or alternatively, sensors 260 may for example also one or more motion sensors, accelerometers etc. to determine a motional state of the battery 210. Additionally or alternatively, sensors 260 may comprise a 3D sensor. The high-voltage capacitor 220 may be the capacitor delivering the energy pulses for the cardioverter and/or defibrillator treatment of the heart. The control unit 240 may be adapted to control the reforming of the high-voltage capacitor 220. The control unit 240 may be connected to the charging circuitry 230, the time unit 250 and/or the orientation sensor 260. The time unit 250 may comprise a timer, timing circuit, counter, and/or a clock generator etc.

The time unit 250 may signal in regular periods, e.g. of about 30-180 days, or about 60-120 days, or about 90 days, to the control unit 240 the necessity to perform a reforming process of the high-voltage capacitor 220. At least in part based thereon, the control unit 240 may switch on and/or analyze data of the orientation sensor 260. The control unit 240 may activate the charging circuitry 230 for reforming the high-voltage capacitor 220 and/or otherwise start the reforming process (only) if the battery 210 and/or defibrillator 200 and/or the patient is determined to be in a favorable orientation and/or motional state for minimizing battery degradation effects resulting from reforming. The favorable state may be based on an orientation of the battery 210, wherein the electrolyte drop 150 may not be significantly formed in the second portion 140 of the battery 210, as described in Fig. 1. The reforming process may be performed over the charging circuitry 230 which may charge the high-voltage capacitor 220 with energy provided from the battery 210 in a defined way.

In some examples, the regular periods used by the time unit 250 may be adapted to be slightly shorter than those required for maintaining the high-voltage capacitor 220. This may take into account that there may be additional waiting time until a suitable state of the battery 210 is confirmed and the coupling can then be activated. In some examples, a timer may be started once the regular period outlined above lapses. If a suitable state of the battery 210 cannot be confirmed before the timer lapses, the coupling may be activated anyway to avoid damage to the capacitor.

In a further example, the implantable defibrillator may not necessarily comprise a sensor 260 that would be capable of determining an orientation and/or motional state of the battery 210. The motional state and/or orientation may then otherwise be determined (e.g. using external sources) and/or estimated, as described herein.

In a further example, additionally or alternatively to sensor(s) 260, an orientation and/or motional state of the battery 210 may be estimated by using (only) data of the time unit 250. For example, the activation of the reforming process may be configured to be at a time, when the patient is estimated to be in an upright body orientation and/or in a rest position. This may correspond to the battery 210 being in a favorable state to minimize excessive battery degradation effects resulting from the reforming process. For example, this may occur in specific morning hours (e.g. 9 - 11 am), when most of the patients are assumed to be not sleeping and to be in a rather upright upper body position. Depending on the implantation direction of the battery, it may also be beneficial to signal the activation during night time (e.g. 1 - 4 am), when patients are assumed to be sleeping, i.e. lying down.

For example, time unit 250 might signal the activation for reforming to the control unit 240 not only in regular periods (as outlined above) but in addition also according to a suitable time of the day being reached. Hence, the likelihood of the reforming taking place when the battery 210 is in an unsuitable orientation and/or motional state is reduced. Alternatively, when time unit 250 signals that reforming is required, control unit 240 may activate the coupling once a suitable time of the day is reached. For example, to this end, the control unit 240 may use its own time and/or inquire a corresponding timer of the time unit 250, and/or use any other source (also possibly external).

## Claims

1. Implantable defibrillator (200), comprising:
an energy source (210);
an electrical component (220);
means for activating (240) a coupling (230) between the energy source (210) and the electrical component (220), based at least in part on an orientation and/or a motional state of the energy source (210),
wherein the electrical component is a capacitor.

2. Implantable defibrillator of claim 1, wherein the implantable defibrillator further comprises means for determining (260) the orientation and/or the motional state.

3. Implantable defibrillator of claim 2, wherein the means for determining (260) the orientation and/or the motional state comprises at least one of the following:
an orientation sensor; a position sensor; an activity sensor; a tilt sensor; an inertial sensor; an acceleration sensor; a rotational sensor; an inertial measurement unit; an inertial navigation system; a 3D-sensor.

4. Implantable defibrillator of any one of the preceding claims, wherein the means for activating (240) is configured to activate the coupling (230), based at least in part on:
the determined orientation deviating from a set orientation value by less than a first predetermined threshold; and/or
the determined motional state deviating from a set motional state value by less than a second predetermined threshold.

5. Implantable defibrillator of one of the preceding claims, further comprising means for estimating the orientation and/or the motional state of the energy source based, at least in part, on a time.

6. Implantable defibrillator of one of the preceding claims, further comprising means for receiving information about the orientation and/or the motional state from an external source.

7. Implantable defibrillator of one of the preceding claims, wherein the energy source (210; 100) comprises:
a first portion (120) which is at least partly filled with liquid;
a second portion (140) which comprises at least one connection (130) for the coupling;
wherein the means for activating (240) is configured to activate the coupling (230), based on the orientation and/or the motional state of the energy source (210; 100) being such, that a flow of liquid from the first portion (120) to the second portion (140) is suppressed.

8. Implantable defibrillator of claim 6, wherein the means for activating (240) is further configured to activate the coupling (230) based on the second portion (140) being in an upper part of the energy source (100).

9. Implantable defibrillator of one of the preceding claims, wherein the means for activating (240) is further configured to activate the coupling (230), based at least in part on a predetermined timing.

10. Implantable defibrillator of one of the preceding claims, wherein the electric component (220) comprises at least one capacitor configured to deliver cardioverter/defibrillator pulses, the implantable defibrillator further configured such that the activating of the coupling reforms the capacitor.

11. Method for operating an implantable defibrillator (200), comprising:
activating a coupling (230) between an energy source (210) and an electrical component (220) of the implantable defibrillator, based at least in part on an orientation and/or a motional state of the energy source (210),
wherein the electrical component is a capacitor.

12. Method of claim 11, further comprising determining the orientation and/or the motional state of the energy source (210).

13. Method of claim 11 or 12, further comprising estimating the orientation and/or the motional state of the energy source (210) based on a time.

14. Method of one of claims 11 to13, further comprising receiving information about the orientation and/or the motional state of the energy source (210) from an external source.

15. Computer program comprising instructions to perform a method of one of claims 11 to 14, when the instructions are executed.
